# EUROPEAN PATENT SPECIFICATION

(11) **EP 0 962 196 B1**
(45) Date of publication and mention of the grant of the patent: **28.09.2005**
(21) Application number: 99304229.0
(22) Date of filing: 01.06.1999
(51) Int. Cl.: A61F 2/16

(54) **Intraocular lens**
Intraocularlinse
Lentille intraoculaire

(30) Priority: 01.06.1998 GB 9811746
(43) Date of publication of application: 08.12.1999
(73) Proprietor: Rayner Intraocular Lenses Limited, East Sussex BN3 7AN (GB)
(72) Inventor: Toop, Peter, Rayner Intraocular Lenses, Ltd., Hove, East Sussex BN3 7AN (GB)
(74) Representative: Perry, Robert Edward

(56) References cited:
- EP-A- 0 215 468
- EP-A- 0 766 952
- WO-A-87/01931
- WO-A-97/20523
- WO-A-97/41805
- US-A- 4 787 904
- US-A- 5 133 749

## Description

### Field of the Invention

This invention relates to an intraocular lens.

### Background of the Invention

A typical intraocular lens comprises an optic and two opposed haptics. There are several constraints on the structure of such a lens. For example, while the haptics should maintain the optic centrally in the capsular sac, they should not be so springy as to damage the sac after insertion, in a folded state. Another important factor determining the form of suitable haptics is that they should be designed to resist excessive asymmetrical capsular contraction, while maintaining the optical performance of the lens, by preventing dislocation caused by buckling or twisting of the haptic or "haptic failure". While sufficient resistance can be provided by using rigid material, such a material is usually incompatible with the need to fold the lens, for insertion into the capsular sac following a small surgical incision.

WO-A-9741805 describes an intraocular implant comprising a substantially circular optic and two curved haptic loops. The distal end of each haptic rests on the internal wall of the eye. The optic and haptics are made of the same flexible material. This material has an elastic modulus of between 0.25 and 1 MPa. The width of each haptic decreases from its proximal end to its distal end, such that the ratio between the bending moment variation and the inertia moment variation at different points on the haptic is substantially constant. In a preferred embodiment, this is achieved by providing an aperture in each haptic; the width of the aperture generally decreases towards the distal end of the haptic.

### Summary of the Invention

The present invention is an intraocular lens comprising an optic and two or more curved haptics which can be compressed in the plane of the lens, wherein each haptic is shaped such that, in use, the proximal part of the haptic can be fully compressed prior to compression of the distal part, and wherein the haptics are compressed to provide an essentially elliptical form of the lens.

Whereas in the first stage of compression, the haptic can be bent inwards with respect to a fulcrum defined by its point of attachment to the optic, a different fulcrum is established for the second stage. This may be the result of the haptic's interference with itself, or interference with the optic. There may be more than two stages of compression, and the number may be such that the effect is continuous, i.e. as in a rolling action where the degree of compression is related to distance from the haptic's point of attachment to the optic.

An intraocular lens according to the present invention is such that its overall size can be reduced by compression against a spring-like resistance of the haptic, sufficient to maintain centration in the capsule. Following the second or a further stage of compression, when the distal end of the haptic is in contact with the lens body, the lens is deformed (in profile) to a shape that is highly resistant to twisting or buckling.

In a preferred embodiment, the lens is deformed to an elliptical shape.

### Description of the Invention

The invention will now be described by way of example only with reference to the accompanying drawings, in which:
Figures 1a and 1b are respectively plan and side views of an intraocular lens embodying the present invention;
Figure 2 is a side view of a lens which has undergone haptic failure due to capsular shrinkage; and
Figures 3 and 4 are plan views of the lens of Fig. 1, after respectively the first and second stages of compression.

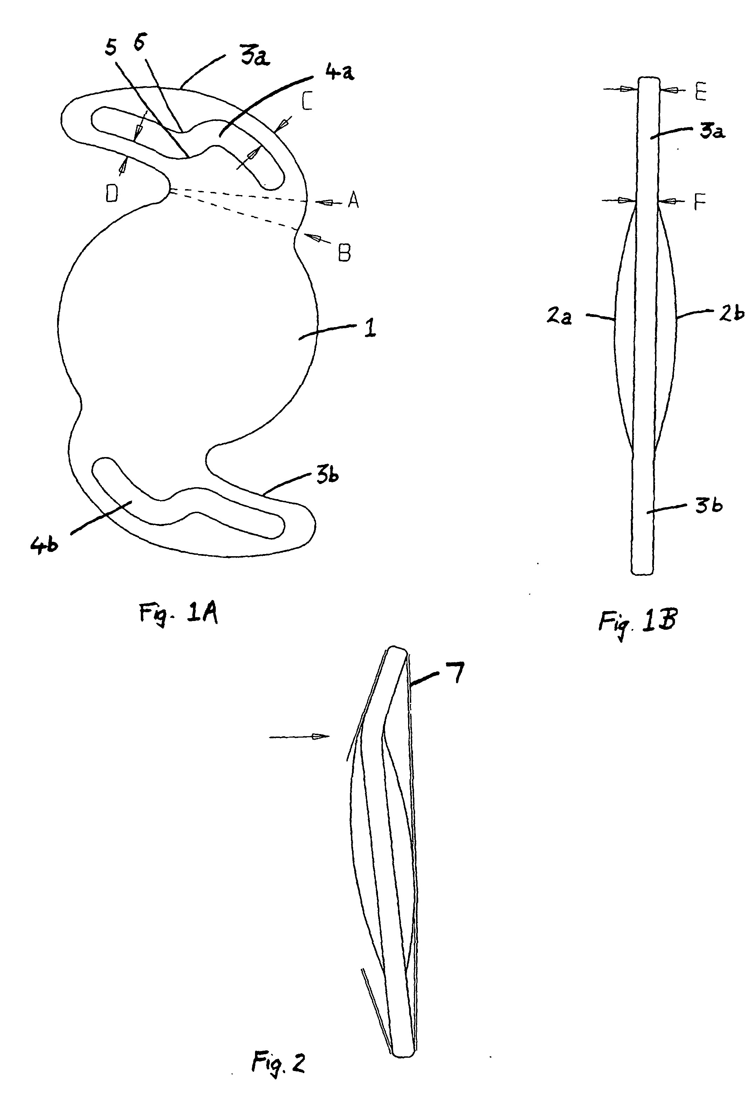

The intraocular lens shown in Fig. 1 comprises a generally circular optic 1 having convex faces 2a and 2b. The lens also comprises haptics 3a and 3b each joined to the optic. Each haptic includes an aperture 4a, 4b.

Each haptic is joined to the optic in generally conventional manner. Fig. la shows dimensions A and B at the joining; typically, A > B.

The outside of the proximal part of each haptic and the inner side of its distal part are defined with respect to the aperture by walls of similar width C and D. Each aperture is of substantially constant width. A more important characteristic of each aperture is its overall shape, including opposed points 5 and 6 which, as described below, can be brought into contact, and effectively define not only a bend in the aperture but also the boundary between proximal and distal parts of the haptic.

As shown in Fig. 2, when there is shrinkage of the capsular sac 7, the haptic is liable to buckle along the line B of Fig. 1. The shape of the haptic and aperture according to the present invention minimise the likelihood of this occurrence.

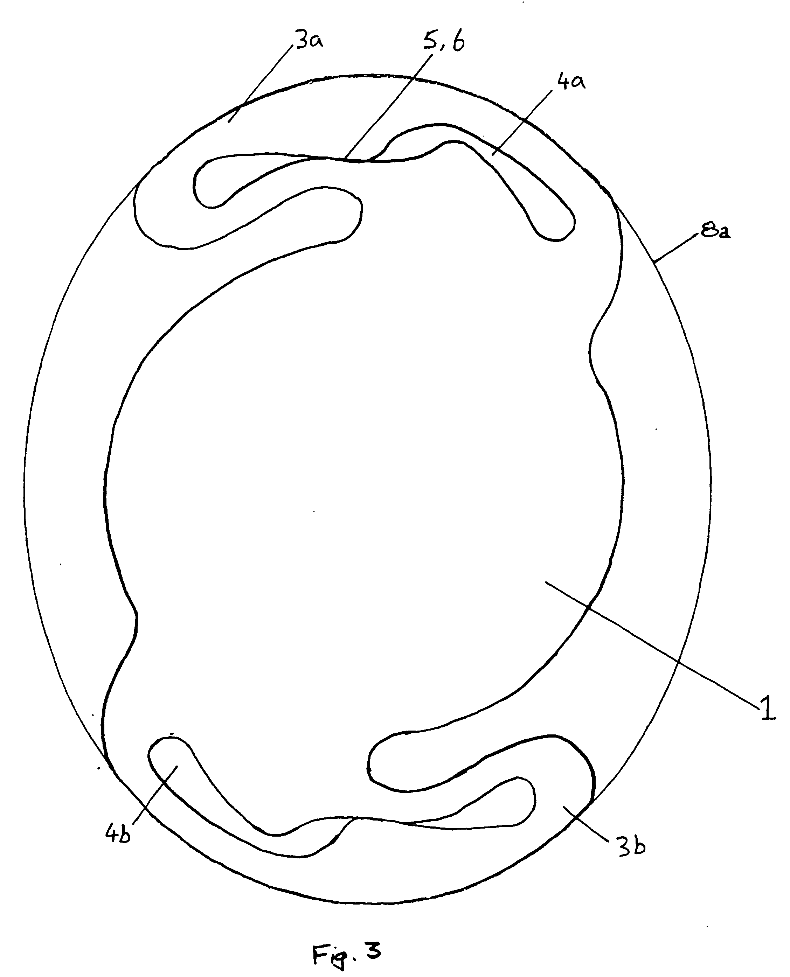

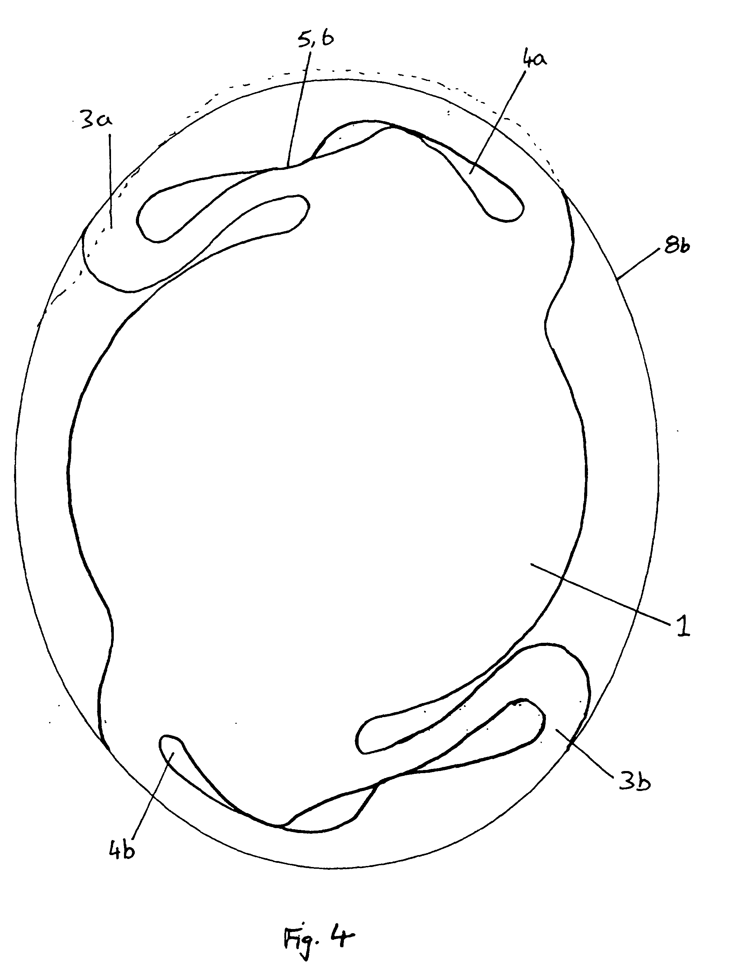

Figs. 3 and 4 each show the essentially elliptical form (8a and 8b) of the lens shown in Fig. 1, when partially and fully compressed, respectively.

More particularly, Fig. 3 shows that initial compression of the haptic leads to abutment of opposite walls of the aperture, bringing points 5 and 6 into contact and thereby defining a proximal part that is fully compressed and a distal part that can undergo further compression. As shown in Fig. 4, such further compression brings the distal end of the haptic substantially into contact with the periphery of the optic, to give an essentially elliptical shape, in plan. This design allows the overall size of the intraocular lens to be reduced, by the spring-like resistance of the haptic, sufficient to maintain centration in the capsule.

Following the first stage of compression, further contraction is prevented in one meridian by interference of one part of the haptic with an adjacent part, and/or with the lens body. Along this meridian, asymmetrical capsular contraction is subsequently prevented. This spring-like resistance of the haptic is then stiffened by the new fulcrum created by the haptic's interference with itself and/or with the lens body. This additional stiffness allows for some further contraction of the capsule along the adjacent meridians, and has the effect of preventing the contraction force along the fixed 10 mm meridian reaching a level sufficient to buckle the haptic. Ultimately, under severe capsular contraction, the distal haptic ends reached the lens body, at which point the lens is deformed (in profile) to the illustrated elliptical shape. This is highly resistant to twisting or buckling.

By way of example, initial contraction may be to dimensions of 10 mm x 8 mm (see Fig. 3). Further contraction gives dimensions of 9.5 mm x 7.5 mm (see Fig. 4).

The thickness of each haptic (e.g. the dimension E shown in Fig. 1b) may be greater than that of conventional lenses. For example, haptics of conventional lenses may be 0.3 mm thick. Each haptic in the present invention may be at least 0.6 mm thick. Such thicker haptics may help to reduce or avoid damage to the lens when it is inserted using a typical lens applicator. Thicker haptics may also advantageously separate anterior and posterior capsule surfaces, which can reduce migration and proliferation of lens epithelial cells from the anterior capsule surface onto the posterior capsule surface.

## Claims

1. An intraocular lens comprising an optic (1) and two or more curved haptics (3a, 3b) which can be compressed, in the plane of the lens, wherein each haptic is shaped such that, in use, the proximal part of the haptic can be fully compressed prior to compression of the distal part; and wherein the haptics are compressed to provide an essentially elliptical form (8b) of the lens.

2. A lens according to claim 1, wherein each haptic includes an aperture (4a, 4b) of which opposed points (5, 6) are brought into contact, during compression of the proximal part.

3. A lens according to claim 1 or claim 2, wherein compression of the proximal part and/or the distal part is essentially continuous, full compression being reached gradually from the proximal end towards the distal end of each haptic.

4. A lens according to any preceding claim, wherein each haptic is at least 0.6mm thick.

## Patentansprüche

1. Intraokulare Linse mit einer Optik (1) und zwei oder mehreren gekrümmten Haptiken (3a, 3b), die in der Ebene der Linse komprimiert werden können, wobei jede Haptik derart geformt ist, dass während der Anwendung der proximale Teil der Haptik vollständig komprimiert werden kann vor der Komprimierung des distalen Teils; und wobei die Haptiken komprimiert werden, um eine im wesentlichen elliptische Form (8b) der Linse zu ergeben.

2. Linse nach Anspruch 1, bei der jede Haptik eine Apertur (4a, 4b) aufweist, von der gegenüberliegende Punkte (5, 6) während der Kompression des proximalen Teils in Kontakt gebracht werden.

3. Linse nach Anspruch 1 oder Anspruch 2, bei der die Kompression des proximalen Teils und/oder distalen Teils im Wesentlichen kontinuierlich ist, wobei eine vollständige Kompression allmählich vom proximalen Ende hin zum distalen Ende jeder Haptik erreicht wird.

4. Linse nach irgendeinem vorhergehenden Anspruch, bei der jede Haptik mindestens 0,6 mm dick ist.

## Revendications

1. Lentille intraoculaire comportant une partie optique (1) et deux ou plus de deux parties haptiques courbées (3a, 3b) qui peuvent être comprimées, dans le plan de la lentille, dans laquelle chaque partie haptique est configurée de manière que, lors de l'utilisation, la portion proximale de la partie haptique puisse être complètement comprimée avant une compression de la portion distale ; et dans laquelle les parties haptiques sont comprimées de façon à donner une forme essentiellement elliptique (8b) à la lentille.

2. Lentille selon la revendication 1, dans laquelle chaque partie haptique présente une ouverture (4a, 4b) dont des points opposés (5, 6) sont amenés en contact pendant une compression de la portion proximale.

3. Lentille selon la revendication 1 ou la revendication 2, dans laquelle une compression de la portion proximale et/ou de la portion distale est essentiellement continue, une compression complète étant atteinte progressivement depuis l'extrémité proximale vers l'extrémité distale de chaque partie haptique.

4. Lentille selon l'une quelconque des revendications précédentes, dans laquelle chaque partie haptique a une épaisseur d'au moins 0,6 mm.
